# EUROPEAN PATENT SPECIFICATION

(11) **EP 4 101 923 B1**
(45) Date of publication and mention of the grant of the patent: **11.12.2024**
(21) Application number: 21751215.1
(22) Date of filing: 01.02.2021
(51) Int. Cl.: C12N 1/16, C12Q 1/04, C40B 30/06

(54) **USE OF A SCREENING CULTURE MEDIUM AND SCREENING METHOD FOR CANDIDA AURIS**
VERWENDUNG EINES SCREENING-KULTURMEDIUMS UND SCREENING-VERFAHREN FÜR CANDIDA AURIS
UTILISATION D'UN MILIEU DE CULTURE DE CRIBLAGE ET PROCÉDÉ DE CRIBLAGE POUR CANDIDA AURIS

(30) Priority: 05.02.2020 JP 2020017999
(43) Date of publication of application: 14.12.2022
(73) Proprietor: Teikyo University, Tokyo 173-8605 (JP)
(72) Inventor: MAKIMURA Koichi, Tokyo 173-8605 (JP); TAMURA Takashi, Tokyo 173-8605 (JP)
(74) Representative: Cabinet Becker et Associés
(86) International application number: PCT/JP2021/003524
(87) International publication number: WO 2021/157521

(56) References cited:
- WO-A1-2019/208691
- ANONYMOUS: "ID 32 C - Identification system for yeasts", 1 July 2011 (2011-07-01), pages 1 - 9, XP093103982, Retrieved from the Internet <URL:https://www.mediray.co.nz/media/15793/om_biomerieux_test-kits_ot-32200_package_insert-32200.pdf> [retrieved on 20231121]
- SATOH KAZUO ET AL: "Candida auris sp. nov., a novel ascomycetous yeast isolated from the external ear canal of an inpatient in a Japanese hospital", MICROBIOLOGY AND IMMUNOLOGY, vol. 53, no. 1, 1 January 2009 (2009-01-01), JP, pages 41 - 44, XP055819069, ISSN: 0385-5600, DOI: 10.1111/j.1348-0421.2008.00083.x
- JOHN OSEI SEKYERE: "Candida auris : A systematic review and meta-analysis of current updates on an emerging multidrug-resistant pathogen", MICROBIOLOGYOPEN, vol. 7, no. 4, 1 August 2018 (2018-08-01), pages e00578, XP055715230, ISSN: 2045-8827, DOI: 10.1002/mbo3.578
- SHARON TSAY ET AL: "Approach to the Investigation and Management of Patients With Candida auris, an Emerging Multidrug-Resistant Yeast", CLINICAL INFECTIOUS DISEASES, vol. 66, no. 2, 17 August 2017 (2017-08-17), US, pages 306 - 311, XP055493986, ISSN: 1058-4838, DOI: 10.1093/cid/cix744
- NAKIMURA KOICHI: "Detection is rare but you should be known bacteria and fungus, Fungus Candida auris", CLINICAL MICROBIOLOGY, vol. 46, no. 5, 30 November 2018 (2018-11-30), JP , pages 441 (057) - 443 (059), XP009538435, ISSN: 0910-7029
- DEVADAS SUGANTHI MARTENA, BALLAL MAMATHA, PRAKASH PERALAM YEGNESWARAN, HANDE MANJUNATH H, GEETHA V, BHAT, MOHANDAS VINITHA: "Auxanographic Carbohydrate Assimilation Method for Large Scale Yeast Identification", JOURNAL OF CLINICAL AND DIAGNOSTIC RESEARCH, vol. 11, no. 4, April 2017 (2017-04-01), pages DC01 - DC03, XP055847587
- DU MENGQIAN, HU WEIMIN, TAMURA TAKASHI, ALSHAHNI MOHAMED MAHDI, SATOH KAZUO, YAMANISHI CHIAKI, NAITO TOSHIO, MAKIMURA KOICHI: "Investigation of the Physiological, Biochemical and Antifungal Susceptibility Properties of Candida auris", MYCOPATHOLOGIA, vol. 186, no. 2, 21 January 2021 (2021-01-21), pages 189 - 198, XP037447590, DOI: https://doi.org/10.1007/s11046-020-00526-w.
- ELBARADEI A. ET AL.: "A decade after the emergence of Candida auris: what do we know?", EUROPEAN JOURNAL OF CLINICAL MICROBIOLOGY & INFECTIOUS DISEASES, vol. 39, no. 9, 15 April 2020 (2020-04-15), pages 1617 - 1627, XP037219665

## Description

### [Technical Field]

The present invention relates to a screening culture medium and a screening method which enables the identification and screening of Candida auris.

Priority is claimed on Japanese Patent Application No. 2020-17999, filed February 5, 2020.

### [Background Art]

There are more than 400 species Candida, known as causative of candidiasis, of which 20 of them are clinically important. Many of them have similar morphological and biochemical properties and are generally difficult to be discriminated.

Studies have been carried out on a method of identifying species with respect to the existing species of Candida. For example, Patent Document 1 discloses a culture medium for simple identification of Candida, which is obtained by adding 2,3,5-triphenyltetrazolium chloride (TTC) and yeast extract to a dye-added Sabouraud's culture medium. This culture medium for simple identification of Candida is intended to be a culture medium for carrying out discrimination between two species of Candida, Candida albicans and Candida glabrata, according to the colony color tone by using the difference in redox ability of these species on the culture medium.

Patent Document 2 discloses a method of detecting the presence of a specific microorganism strain in a culture medium, which is a method in which at least one chromogen that is a substrate for the enzyme of the strain and at least one compound of a high concentration, selected from carbohydrates, are added to a culture medium, and after the hydrolysis of the chromogen, an induced color different from the basic color of the chromophore is obtained. This method is intended to be one in which, for an enzyme that is produced by each of the bacteria including Candida albicans and Candida tropicalis, two chromogens, which are substrates of the enzyme, are added to a culture medium, and after the hydrolysis of the chromogens, colors different from the basic color of the chromophore are obtained to carry out distinguishment between the two strains.

### [Citation List]

### [Patent Documents]

[Patent Document 1]
   Japanese Unexamined Patent Application, First Publication No. H06-78793
[Patent Document 2]
   Published Japanese Translation No. H09-500790 of the PCT international Publication

Osei Sekyere characterizes the multi-drug resistant pathogen Candida auris and details methods for identifying it (MicrobiologyOpen 2018 7:e578).

### [Summary of Invention]

### [Technical Problem]

Among the Candida, Candida auris, discovered and named by the inventors of the present invention have acquired multiple drug resistance and exhibited a high mortality rate, which has posed a global threat. There is a particular need for a means for conveniently and quickly examining whether or not this species, among the Candida, is present in a specific environment (for example, a certain closed space such as a hospital room).

For identifying Candida auris with respect to other species of Candida, the identification can be carried out for example, by analyzing the genes. However, this analysis requires equipment which may be inconvenient, and requires time and cost as well.

In the detection technique using the ability to degrade an enzyme substrate such as Patent Document 2 described above, it is possible to carry out detection with relatively convenient equipment and operation in a short culture time, and in the above technique and a detection method applying the above technique, other Candida such as Candida albicans and Candida tropicalis can be detected. However, according to the above technique, a method of distinguishing Candida auris from other Candida has not been found, and thus Candida auris cannot be detected by this technique.

For this reason, a technique for conveniently and quickly examining the existence of Candida auris has not yet been found.

The present invention has been made in consideration of the above circumstances, and an object thereof is to provide a screening culture medium that enables a convenient and quick examination for the presence of Candida auris, and a screening method using the screening culture medium.

### [Solution to Problem]

In order to solve the above object, the present invention has the following aspects.
[1] Use of a screening culture medium for screening Candida auris based on a positive or negative ability of Candida auris to degrade an enzyme substrate, wherein the medium comprises an enzyme substrate, wherein the enzyme substrate includes raffinose and xylose, wherein each of raffinose and xylose is labeled such that a different color is developed in a case of being degraded, and wherein the microorganism is determined to be Candida auris, in a case where the microorganism has a positive ability to degrade raffinose, and a negative ability to degrade xylose.
[2] The use of the above screening culture mediums, in which the Candida auris is an East Asian strain of Candida auris, and the enzyme substrate further includes N-acetylglucosamine.
[3] The use of the above screening culture mediums, in which the Candida auris is an East Asian strain of Candida auris, and the enzyme substrate further includes potassium gluconate.
[4] The use of the above screening culture mediums, in which the enzyme substrate further includes glycerol.
[5] The use of the above screening culture mediums, in which the enzyme substrate further includes D-glucosamine hydrochloride.
[6] A method for screening the microorganism Candida auris based on a positive or negative ability of Candida auris to degrade an enzyme substrate comprising:- a step of inoculating a sample containing a microorganism in a screening culture medium containing an enzyme substrate, said enzyme substrate including raffinose and xylose;- a step of culturing the microorganism contained in the sample in the screening culture medium; and- a step of screening Candida auris as the microorganism by detecting Candida auris in the screening culture medium;wherein the screening culture medium enables detection of the presence of the microorganism Candida auris based on an ability of the microorganism to degrade the enzyme substrate, by allowing each enzyme substrate to develop a different color, andwherein the microorganism is determined to be Candida auris, in a case where the microorganism has a positive ability to degrade raffinose, and a negative ability to degrade xylose.
[7] Any of the above screening methods, in which in a case where the enzyme substrate further includes N-acetylglucosamine, and the microorganism has a negative ability to degrade N-acetylglucosamine, the microorganism is determined to be an East Asian strain of Candida auris.
[8] Any of the above screening methods, in which in a case where the enzyme substrate further includes potassium gluconate, and the microorganism has a negative ability to degrade potassium gluconate, the microorganism is determined to be an East Asian strain of Candida auris.
[9] Any of the above screening methods, in which in a case where the enzyme substrate further includes glycerol, and the microorganism has an ability to degrade glycerol by more than 0*%* and 20*%* or less, which is negative, the microorganism is determined to be an East Asian strain of Candida auris.
[10] Any of the above screening methods, in which in a case where the enzyme substrate further includes glycerol, and the microorganism has a positive ability to degrade glycerol, the microorganism is determined to be a South American, African, or South Asian strain of Candida auris.
[11] Any of the above screening methods, in which in a case where the enzyme substrate further includes D-glucosamine hydrochloride, and the microorganism has a negative ability to degrade D-glucosamine hydrochloride, the microorganism is determined to be an East Asian strain of Candida auris.
[12] Any of the above screening methods, in which in a case where the enzyme substrate further includes D-glucosamine hydrochloride, and the microorganism has an ability to degrade D-glucosamine hydrochloride by 80*%* or more and less than 100*%*, which is positive, the microorganism is determined to be a South American strain of Candida auris.
[13] Any of the above screening methods, in which in a case where the enzyme substrate further includes D-glucosamine hydrochloride, and the microorganism has a positive ability to degrade D-glucosamine hydrochloride, the microorganism is determined to be an African or South Asian strain of Candida auris.
[14] Any of the above screening methods, in which in the step of culturing the microorganism, the culture is carried out at 35°C or higher.

### [Advantageous Effects of Invention]

According to the present invention, it is possible to obtain a screening culture medium that enables a convenient and quick examination for the presence of Candida auris, and a screening method using the screening culture medium.

### [Description of Embodiments]

Hereinafter, use of a screening culture medium and a screening method according to the present invention will be described with reference to embodiments. However, the present invention is not limited to the following embodiments.

### (Use of a screening culture medium)

The present invention relates to the use of a screening culture medium for screening Candida auris based on a positive or negative ability of Candida auris to degrade an enzyme substrate, wherein the medium comprises an enzyme substrate, wherein the enzyme substrate includes raffinose and xylose, wherein each of raffinose and xylose is labeled such that a different color is developed in a case of being degraded, and wherein the microorganism is determined to be Candida auris, in a case where the microorganism has a positive ability to degrade raffinose, and a negative ability to degrade xylose.

Screening broadly refers to an operation of sorting out a microorganism. It broadly refers to an operation of sorting out a sample to be screened containing a microorganism, from the stage of the presence or absence of the possibility, by the presence or absence of a certain microorganism. The screening also refers to a step of obtaining more information for determining the possibility that a sample contains a certain microorganism (for example, determining the possibility from the results of a plurality of screenings).

The screening operation may be carried out one time or a plurality of times. Alternatively, the same screening operation may be carried out a plurality of times, and this series of operations may be collectively referred to as screening.

The screening culture medium is used for screening Candida auris.

There are various strains of Candida auris, which are classified into clades such as a South American clade, an African clade, an East Asian (Japanese and Korean) clade, and a South Asian (Indian) clade, depending on the origin thereof. Using the screening culture medium according to the present embodiment, any strain belonging to any clade can be screened as long as it is Candida auris.

As such a screening culture medium, it is possible to use, for example, a known enzyme substrate culture medium. The enzyme substrate culture medium is a culture medium in which an enzyme substrate labeled with a chromogen (a color-developing substance) is contained, where the enzyme substrate is a substrate specifically metabolized (degraded and assimilated) by a target microorganism (a bacterial species). In a case where an enzyme substrate is incorporated and metabolized by a target bacterial species, a chromogen dissociates and the condensed chromogen develops a color (is colored). As a result, it is possible to screen a bacterial species having an ability to degrade the enzyme substrate.

As the chromogen with which an enzyme substrate is labeled, it is possible to use, for example, a compound that releases by hydrolysis two different chromophores that are capable of causing a coupling reaction. As such a compound, it is possible to use, for example, an indoxyl derivative. In a case where a culture medium contains a plurality of enzyme substrates, it is preferable to use a chromogen that exhibits a different color for each enzyme substrate, for example, a chromogen of a different compound.

The screening culture medium according to the present embodiment contains raffinose (RAF) and xylose (XYL) among the enzyme substrates.

Candida auris exhibits a positive ability to degrade raffinose and a negative ability to degrade xylose. Specifically, all of the 44 strains of Candida auris, including each of the above clades, exhibit positivity to raffinose (positive rate: 100*%*) and negativity to xylose (positive rate: 0*%*). As a result, in a case where a microorganism cultured in the screening culture medium of the present embodiment exhibits a positive ability to degrade raffinose and a negative ability to degrade xylose, there is a high possibility that such a microorganism is Candida auris. In addition, the combination of the positive ability to degrade raffinose and the negative ability to degrade xylose is not found in major pathogens other than Candida auris. As a result, the culture medium is used for screening for Candida auris.

The screening culture medium is an enzyme substrate culture medium, and contains raffinose and xylose each labeled with a chromogen that exhibits a different color at the time of degradation.

According to the above configuration, it is possible to carry out screening such that, among the colonies of microorganisms cultured on the solid culture medium, a colony that exhibits a color obtained in a case where raffinose is degraded and does not exhibit a color obtained in a case where xylose is degraded is Candida auris, and a colony that exhibits only the color obtained in a case where xylose is degraded or both the colors obtained in a case where raffinose and xylose are degraded is not Candida auris.

### (Culture medium containing other substrates)

The enzyme substrate contained in the screening culture medium of the present embodiment may further contain another enzyme substrate in addition to raffinose and xylose. As the enzyme substrate, enzyme substrates that are used in the related art for identifying a species can be appropriately selected. As a preferred example thereof, the screening culture medium may further contain N-acetylglucosamine (NAG), potassium gluconate (GNT), glycerol (GLY), or D-glucosamine hydrochloride (GLN). As will be described later regarding the screening method, in a case where the screening culture medium contains these enzyme substrates in addition to raffinose and xylose, it is possible to obtain the information on which clade of strain a microorganism belongs to, in addition to the information on whether it is Candida auris.

In a case where the screening culture medium is the enzyme substrate culture medium described above and contains further enzyme substrate other than raffinose and xylose, the other enzyme substrate is preferably labeled to develop a color different from those of raffinose and xylose. According to this above configuration, it is possible to obtain the information on which clade of strain a cultured microorganism belongs to, concurrently with the information on whether or not it is Candida auris, based on the coloration that exhibits an ability to degrade each of raffinose, xylose, and another enzyme substrate (such as N-acetylglucosamine).

### (Other configurations)

In a case where the screening culture medium of the present embodiment is an enzyme substrate culture medium, other configurations thereof may be those that are used for the existing culture medium. For example, components can be appropriately selected from the compositions of known enzyme substrate culture mediums, for example, a color-developing medium. The enzyme substrate culture medium may contain nutrients such as peptone, yeast extract, sugar, minerals, and vitamins. As the nutrient, peptone is suitably used. The enzyme substrate culture medium may contain a thickening agent, a pH-adjusting agent, or the like. Further, it may contain an additive for improving the color development of the enzyme substrate, for example, a redox reagent.

The enzyme substrate culture medium may contain an antibacterial agent for suppressing the growth of microorganisms other than the target microorganism for screening. Examples of the antibacterial agent include an aminoglycoside-based antibiotic, a broad-spectrum antibiotic such as chloramphenicol or tetracycline, and cephalosporins or penicillin. Chloramphenicol is suitably used as an antibiotic for screening Candida auris.

The enzyme substrate culture medium may take an existing form as appropriate, such as liquid, semi-fluid, or solid; however, for example, in a case where a solid culture medium, particularly a flat plate solid culture medium (having a plate shape) is used, culture and detection are easy. A solidifying agent that is used in the related art, such as agar, carrageenan, or locust bean gum can be used to form a solid culture medium, and in the present embodiment, agar is used.

It is to be noted that a case where the screening culture medium contains a plurality of enzyme substrates may be a case of a combination of a plurality of culture media containing one or more of the plurality of enzyme substrates, in addition to a case where one culture medium contains all the enzyme substrates. For example, in a case where one kind of microorganism has been isolated in advance and a sample contains only that microorganism, and in a case where the information on whether the microorganism is Candida auris or the information on other clades of the microorganism is to be obtained, the present embodiment also includes an aspect in which the sample is cultured in different culture media each containing a different enzyme substrate and the information on the ability to degrade each enzyme substrate is obtained.

### (Screening method)

### (Screening of Candida auris with culture medium containing RAF and XYL)

In a further aspect of the invention a method for screening the microorganism Candida auris is provided based on a positive or negative ability of Candida auris to degrade an enzyme substrate, the method comprising:- a step of inoculating a sample containing a microorganism in a screening culture medium containing an enzyme substrate, said enzyme substrate including raffinose and xylose;- a step of culturing the microorganism contained in the sample in the screening culture medium; and- a step of screening Candida auris as the microorganism by detecting Candida auris in the screening culture medium;wherein the screening culture medium enables detection of the presence of the microorganism Candida auris based on an ability of the microorganism to degrade the enzyme substrate, by allowing each enzyme substrate to develop a different color, andwherein the microorganism is determined to be Candida auris, in a case where the microorganism has a positive ability to degrade raffinose, and a negative ability to degrade xylose.

According to the screening method of the present embodiment, it is possible to carry out screening for whether or not a sample contains Candida auris, where the sample contains a plurality of microorganisms, or it is possible to carry out screening for whether or not a certain microorganism has a high possibility of being Candida auris.

As the sample containing a plurality of microorganisms, a sample collected from a specific space in which microorganisms might be present can be conceived.

Specifically, the screening method includes a step of inoculating a sample in a screening culture medium containing the enzyme substrate; a step of culturing a microorganism contained in the sample in the screening culture medium; and a step of detecting the microorganism in the screening culture medium.

For example, a method of carrying out screening for whether a sample containing a plurality of microorganisms contains Candida auris by using the above-described enzyme substrate culture medium will be described.

First, a step of inoculating a sample in a screening culture medium containing the enzyme substrate is carried out. Inoculation may be appropriately carried out according to the cell culture method in the related art.

Then, a step of culturing the microorganisms contained in the sample is carried out. Specifically, by culturing the microorganisms in the enzyme substrate culture medium inoculated with the sample, colonies of the microorganisms are formed on the enzyme substrate culture medium.

The conditions for carrying out culture in the enzyme substrate culture medium can be appropriately selected from the conditions for culturing microorganisms, known in the related art. In the present embodiment, it is preferable to carry out the culture at 35°C or higher. In a case of carrying out the culture at 35°C or higher, it is possible to culture pathogenic bacteria that grow under the conditions close to the human body temperature, thereby eliminating microorganisms that do not grow at lower temperatures. It is also preferable to carry out the culture at a temperature higher than 40°C and lower than 42°C. Since Candida auris can grow even at 41°C, it is possible to eliminate other microorganisms that grow at a temperature up to 40°C by carrying out culture under these temperature conditions.

The culture time can be appropriately selected from the conditions for culturing microorganisms, known in the related art; however, the culture is carried out for, for example, 24 hours or more and preferably 48 hours or more. In a case of carrying out culture for the above time, it is possible to form colonies sufficiently to the extent that coloration can be checked.

Then, a step of detecting the microorganisms in the screening culture medium is carried out. In the present embodiment, in a case of checking the coloration of the colonies of the microorganisms formed on the enzyme substrate culture medium, it is possible to carry out screening for whether the microorganism of the colony is Candida auris. Regarding the checking of the coloration, it is sufficient to visually check whether or not the color of the color-developing body with which the enzyme substrate is labeled is developed for each colony of the microorganism.

In the above-described enzyme substrate culture medium, a screening culture medium is used, where the screening culture medium enables the detection of the ability of the microorganism to degrade the enzyme substrate, by allowing each enzyme substrate to develop a different color. That is, in the enzyme substrate culture medium of the present embodiment, the enzyme substrates raffinose and xylose are each labeled to develop a different color in a case of being degraded. As described above, Candida auris exhibits an ability to degrade raffinose but does not exhibit an ability to degrade xylose. As a result, it can be determined that among the colonies of microorganisms cultured on the above culture medium, a colony that exhibits a color that is developed in a case where raffinose is degraded and does not exhibit a color that is not exhibited in a case where xylose is degraded is Candida auris. In a case where a sample containing a plurality of microorganisms has been inoculated in an enzyme substrate culture medium, and in a case where a colony that shows this coloration is included in the cultured colonies, it can be determined that the sample contains Candida auris.

### (Screening of bacterial clade with culture medium containing other substrates)

In a case where the screening culture medium of the present embodiment further contains glucosamine (NAG), among the Candida auris strains, a strain of the East Asian clade exhibits negativity to N-acetylglucosamine, and strains of the South American, African, and South Asian clades exhibit positivity thereto. As a result, in a case of using this culture medium, it is possible to screen a strain of the East Asian clade of Candida auris.

Regarding the clades of Candida auris, it is known that other clades among these clades are more pathogenic than the East Asian clade. Regarding the East Asian clade, it is conceived that a fatal infection does not necessarily occur since the infection is limited to local infection that is basically limited to the inner ear and the outer ear.

That is, in a case of carrying out screening for whether or not a certain microorganism is Candida auris and also on whether or not it belongs to the East Asian clade, it is possible to obtain the information on the risk of the microorganism, or the sample containing the microorganism, or the environment in which the sample has been collected.

Further, in a case where the screening culture medium of the present embodiment further contains potassium gluconate (GNT), among the Candida auris strains, a strain of the East Asian clade exhibits negativity to N-acetylglucosamine, and strains of the South American, African, and South Asian clades exhibit positivity thereto. As a result, in a case of using this culture medium, it is possible to obtain the information for screening a strain of the East Asian clade of Candida auris.

In addition, in a case where the screening culture medium of the present embodiment further contains glycerol (GLY), among the Candida auris strains, only some of strains (roughly, more than 0*%* and less than 20*%*) of the East Asian clade exhibit positivity to glycerol, and strains of the South American, African, and South Asian clades exhibit positivity thereto. As a result, in a case of using this culture medium, it is possible to obtain the information for screening a strain of the East Asian clade of Candida auris and other strains.

In addition, in a case where the screening culture medium of the present embodiment further contains D-glucosamine hydrochloride (GLN), among the Candida auris strains, a strain of the East Asian clade exhibits negativity to D-glucosamine hydrochloride, strains of the African and South American clades exhibit positivity thereto, and a large number of strains (roughly, more than 80*%* and less than 100*%*) of the South Asian clades exhibit positivity thereto. As a result, in a case of using this culture medium, it is possible to obtain the information for screening a strain of the East Asian clade of Candida auris and other strains.

In a case where the screening culture medium is the enzyme substrate culture medium described above and contains a further enzyme substrate other than raffinose and xylose, and furthermore, the other enzyme substrate is labeled to develop a color different from those of raffinose and xylose, it is possible to carry out screening for whether or not a cultured microorganism is Candida auris and concurrently for whether or not it is the East Asian clade, based on the coloration that exhibits an ability to degrade each of raffinose, xylose, and another enzyme substrate (such as N-acetylglucosamine).

The screening based on such coloration can also be carried out by appropriately combining two or more substrates each providing different coloration. For example, in a case where a screening target is positive to two or more substrates, two or more types of coloration are exhibited, and a microorganism colony exhibits a color resulting from the mixed coloration, whereby it is possible to obtain the information on which substrate reacts with the screening target, that is, which strain of clade among Candida auris strains is included, based on the color tone and intensity of the colony.

### (Effect of present embodiment)

The screening culture medium and screening method according to the present embodiment enables a convenient and quick examination for the presence of Candida auris. Specifically, for a sample containing a plurality of microorganisms, for example, a sample collected from a specific space, it is possible to carry out screening for the presence of Candida auris with a convenient operation of just inoculating and culturing the sample in a flat plate solid culture medium and in a short time of about 24 to 48 hours. For inoculation and culturing, it suffices that such equipment that enables culture in a flat plate solid culture medium is provided. Furthermore, the screening of microorganisms is carried out by only visual observation of the coloration, and thus special equipment or instrument is not required. Screening with a flat plate solid culture medium can be carried out at a very low cost as compared with gene analysis and the like.

### (Application example of present embodiment)

As the sample to be screened in the present embodiment, for example, a sample collected from a specific indoor or outdoor space can be conceived. Examples of the specific space include places where Candida auris should be detected, and they include rooms in a hospital, particularly operating rooms or hospital rooms. In addition, those from various facilities or welfare facilities with elderly people and sick people can be used. In a case where the detection is carried out at an airport or a customhouse, it can be used for so-called border control measures.

The screening culture medium and the method of the present embodiment can be used to obtain information on the presence/absence of Candida auris or any clade of bacteria by combining a screening operation a plurality of times.

In addition, the screening culture medium and the method of the present embodiment can be used in combination with other means. For example, the screening culture medium and the method of the present embodiment can be used as a pre-stage screening for narrowing down the target to be subjected to further detailed gene analysis. Since the screening method of the present embodiment can be carried out conveniently and quickly, it is particularly effective in a case where the number or the like of samples to be screened (specific spaces where the presence is examined), clades, or the like is large.

### (Other embodiments)

The screening method of the present embodiment can also be used to carry out screening for one kind of microorganism on whether the bacterium is Candida auris. In that case, it is also possible to carry out screening using an enzyme substrate culture medium containing the above-described raffinose and xylose or containing another enzyme substrate. In addition, the present embodiment also includes an aspect in which the culture is carried out in different culture media each containing a different enzyme substrate and the information on the ability to degrade each enzyme substrate is obtained.

For the enzyme substrate culture medium, a plate-shaped culture medium is easy to use for culturing microorganisms; however, another form may be used. For example, a culture medium may be formed into a thin film shape. Since it is not difficult to simply install a film-shaped culture medium in various places and culture as is for carrying out screening, it is possible to install the film-shaped culture medium in the above-described specific space where Candida auris should be detected.

### [Examples]

Hereinafter, Examples will be described. It is to be noted that the present invention is not limited to the following Examples.

### (Verification of enzyme substrate specific to Candida auris)

The ability to degrade each enzyme substrate was examined for a plurality of bacterial strains including Candida auris. As Candida auris, 44 strains shown in Table 1 (as the species, 10 strains of the South American clade, 10 strains of the African clade, 10 strains of the East Asian clade, and 14 strains of the South Asian clade) were used. Other strains were selected from among the representative pathogenic yeast bacterial strains in Japan. The strains used as strains other than Candida auris are shown in Table 2.

As a kit that can identify the degradation ability as a positive rate, a plate of culture identification/fungus kit ID 32C API (Sysmex BioMérieux Co., Ltd.) was used for various enzyme substrates including raffinose (RAF), xylose (XYL), N-acetylglucosamine (NAG), potassium gluconate (GNT), glycerol (GLY), and D-glucosamine hydrochloride (GLN) among the enzyme substrates.

Solutions of the strains shown in Tables 1 and 2 were prepared with a suspension medium (a liquid culture medium) according to the protocol and taken into a positive rate detection cup of each enzyme substrate on a plate of ID 32C API and cultured for 48 hours. After culturing, wells that were transparent similar to the control were judged as negative (-), and wells that were opaque as compared with the control were judged as positive (+) by visual determination, and then the obtained positive data were analyzed using the database software 4.0 attached to ID 32C API.

**[Table 1]**

| Strain | | | | | | | | |
|---|---|---|---|---|---|---|---|---|
| Test example | | Clade | RAF | XYL | NAG | GNT | GLY | GLN |
| 1 | C. auris #16 | South American | + | - | + | + | + | + |
| 2 | C. auris #17 | South American | + | - | + | + | + | + |
| 3 | C. auris #18 | South American | + | - | + | + | + | + |
| 4 | C. auris #19 | South American | + | - | + | + | + | + |
| 5 | C. auris #20 | South American | + | - | + | + | + | + |
| 6 | C. auris #21 | South American | + | - | + | + | + | + |
| 7 | C. auris #22 | South American | + | - | + | + | + | + |
| 8 | C. auris #23 | South American | + | - | + | + | + | + |
| 9 | C. auris #24 | South American | + | - | + | + | + | + |
| 10 | C. auris #25 | South American | + | - | + | + | + | + |

| South American positive rate | | | 100 | 0 | 100 | 100 | 100 | 100 |
|---|---|---|---|---|---|---|---|---|
| 11 | C. auris #26 | African | + | - | + | + | + | + |
| 12 | C. auris #27 | African | + | - | + | + | + | + |
| 13 | C. auris #28 | African | + | - | + | + | + | + |
| 14 | C. auris #29 | African | + | - | + | + | + | + |
| 15 | C. auris #30 | African | + | - | + | + | + | + |
| 16 | C. auris #31 | African | + | - | + | + | + | + |
| 17 | C. auris #32 | African | + | - | + | + | + | - |
| 18 | C. auris #33 | African | + | - | + | + | + | + |
| 19 | C. auris #34 | African | + | - | + | + | + | + |
| 20 | C. auris #35 | African | + | - | + | + | + | + |

| African positive rate | | | 100 | 0 | 100 | 100 | 100 | 90 |
|---|---|---|---|---|---|---|---|---|
| 21 | C. auris #43 | East Asian | + | - | - | - | + | - |
| 22 | C. auris #44 | East Asian | + | - | - | - | - | - |
| 23 | C. auris 3540 | East Asian | + | - | - | - | - | - |
| 24 | C. auris 3541 | East Asian | + | - | - | - | - | - |
| 25 | C. auris 3435 | East Asian | + | - | - | - | - | - |
| 26 | C. auris 3436 | East Asian | + | - | - | - | - | - |
| 27 | C. auris 3449 | East Asian | + | - | - | - | - | - |
| 28 | C. auris 643 | East Asian | + | - | - | - | - | - |
| 29 | C. auris 3212 | East Asian | + | - | - | - | - | - |
| 30 | C. auris 3213 | East Asian | + | - | - | - | - | - |

| East Asian positive rate | | | 100 | 0 | 0 | 0 | 10 | 0 |
|---|---|---|---|---|---|---|---|---|
| 31 | C. auris #52 | South Asian | + | - | + | + | + | + |
| 32 | C. auris #53 | South Asian | + | - | + | + | + | + |
| 33 | C. auris 3214 | South Asian | + | - | + | + | + | + |
| 34 | C. auris 3215 | South Asian | + | - | + | + | + | + |
| 35 | C. auris 3216 | South Asian | + | - | + | + | + | + |
| 36 | C. auris 3217 | South Asian | + | - | + | + | + | + |
| 37 | C. auris 3218 | South Asian | + | - | + | + | + | + |
| 38 | C. auris 3219 | South Asian | + | - | + | + | + | + |
| 39 | C. auris 3220 | South Asian | + | - | + | + | + | + |
| 40 | C. auris 3221 | South Asian | + | - | + | + | + | + |
| 41 | C. auris 3222 | South Asian | + | - | + | + | + | + |
| 42 | C. auris 3223 | South Asian | + | - | + | + | + | + |
| 43 | C. auris 3224 | South Asian | + | - | + | + | + | + |
| 44 | C. auris 3225 | South Asian | + | - | + | + | + | + |

| South Asian positive rate | | | 100 | 0 | 100 | 100 | 100 | 100 |
|---|---|---|---|---|---|---|---|---|
| | | | | | | | | |
| Total positive rate | | | 100 | 0 | 77 | 77 | 80 | 75 |

**[Table 2]**

| | RAF-positive rate (*%*) | XYL-positive rate (*%*) | Probability of RAF positivity and XYL negativity (*%*) |
|---|---|---|---|
| Candida albicans 1 | 1 | 98 | 0.00*%* |
| Candida albicans 2 | 0 | 67 | 0.00*%* |
| Candida dubliniensis | 1 | 3 | 1.00*%* |
| Candida famata | 93 | 75 | 23.30*%* |
| Candida glabrata | 0 | 1 | 0.00*%* |
| Candida globosa | 60 | 0 | 60.00*%* |
| Candida guilliermondii | 100 | 99 | 1.00*%* |
| Candida intermedia | 85 | 99 | 0.90*%* |
| Candida kefyr | 99 | 83 | 16.80*%* |
| Candida krusei | 0 | 1 | 0.00*%* |
| Candida lusitaniae | 7 | 94 | 0.40*%* |
| Candida parapsilosis | 1 | 96 | 0.00*%* |
| Candida sake | 2 | 74 | 0.50*%* |
| Candida tropicalis | 9 | 99 | 0.10*%* |
| Cryptococcus neoformans | 81 | 81 | 15.40*%* |
| Rhodotorula minuta | 0 | 86 | 0.00*%* |
| Candida auris | 100 | 0 | 100.00*%* |

As shown in Table 1, all of the 44 strains of 4 clades of Candida auris had a positive ability to degrade RAF and a negative ability to degrade XYL.

Table 2 shows that all of the 44 strains of Candida auris (the bottom row of the table) had a positive ability (100*%*) to degrade RAF and a negative ability (0*%*) to degrade XYL. On the other hand, as shown in the other rows of Table 2, in the pathogenic bacterial strains other than Candida auris, the combination of a positive ability of 100*%* to degrade RAF and a negative ability (positive ability: 0*%*) to degrade XYL was not found.

That is, among the major pathogenic strains only Candida auris had a positive ability of 100*%* to degrade RAF and a negative ability of 100*%* to degrade XYL. It was seen that it is possible to carry out screening for the presence or absence of Candida auris by detecting the ability to degrade RAF and the ability to degrade XYL.

As shown in the results shown in Table 1, among the 44 strains of Candida auris, the East Asian strain was positive to RAF and negative to XYL, as well as 100*%* negative to NAG.

That is, it was seen that in a case of detecting an ability of a Candida auris strain to degrade NAG in addition to RAF and XYL, it is possible to carry out screening for whether it is an East Asian strain or another strain.

Further, as shown in Table 1, the East Asian strain was 0*%* positive to GNT, whereas the strains other than the East Asian were 100*%* positive thereto.

The East Asian clade was 10*%* positive to GLY (that is, only one strain of the 10 East Asian strains was positive). From these results, it is conceived that the strains belonging to the East Asian clade include strains that are positive in a range of about 0 to 20*%* with respect to GLY. On the other hand, strains other than the East Asian were 100*%* positive thereto.

The East Asian clade was 0*%* positive to GLN, whereas the African and South Asian clades were 100*%* positive thereto, and the South American clade was 90*%* positive thereto. From this, it is conceived that the strains belonging to the South Asian clade include strains that are positive in a range of about 80*%* to 100*%* with respect to GLY

That is, a strain having a low positive rate to GLY and a strain negative to GLN can be distinguished from other strains as the East Asian clade, and it is conceived that these combinations can be used for screening judgment of each of the strains.

### [Industrial Applicability]

According to the present invention, it is possible to use a screening culture medium that enables a convenient and quick examination for the presence of Candida auris, and a screening method using the screening culture medium.

## Claims

1. Use of a screening culture medium for screening *Candida auris* based on a positive or negative ability of *Candida auris* to degrade an enzyme substrate,
wherein the medium comprises an enzyme substrate,
wherein the enzyme substrate includes raffinose and xylose,
wherein each of raffinose and xylose is labeled such that a different color is developed in a case of being degraded, and
wherein the microorganism is determined to be *Candida auris*, in a case where the microorganism has a positive ability to degrade raffinose, and a negative ability to degrade xylose.

2. The use of a screening culture medium according to Claim 1, wherein the *Candida auris* is an East Asian strain of *Candida auris*, and the enzyme substrate further includes N-acetylglucosamine.

3. The use of a screening culture medium according to Claim 1 or 2, wherein the *Candida auris* is an East Asian strain of *Candida auris*, and the enzyme substrate further includes potassium gluconate.

4. The use of a screening culture medium according to any one of Claims 1 to 3, wherein the enzyme substrate further includes glycerol.

5. The use of a screening culture medium according to any one of Claims 1 to 4, wherein the enzyme substrate further includes D-glucosamine hydrochloride.

6. A method for screening the microorganism *Candida auris* based on a positive or negative ability of *Candida auris* to degrade an enzyme substrate comprising:
- a step of inoculating a sample containing a microorganism in a screening culture medium containing an enzyme substrate, said enzyme substrate including raffinose and xylose;
- a step of culturing the microorganism contained in the sample in the screening culture medium; and
- a step of screening *Candida auris* as the microorganism by detecting *Candida auris* in the screening culture medium;
wherein the screening culture medium enables detection of the presence of the microorganism *Candida auris* based on an ability of the microorganism to degrade the enzyme substrate, by allowing each enzyme substrate to develop a different color, and
wherein the microorganism is determined to be *Candida auris*, in a case where the microorganism has a positive ability to degrade raffinose, and a negative ability to degrade xylose.

7. The screening method according to Claim 6, wherein in a case where the enzyme substrate further includes N-acetylglucosamine, and the microorganism has a negative ability to degrade N-acetylglucosamine, the microorganism is determined to be an East Asian strain of *Candida auris.*

8. The screening method according to Claim 6, wherein in a case where the enzyme substrate further includes potassium gluconate, and the microorganism has a negative ability to degrade potassium gluconate, the microorganism is determined to be an East Asian strain of *Candida auris.*

9. The screening method according to Claim 6, wherein in a case where the enzyme substrate further includes glycerol, and the microorganism has an ability to degrade glycerol by more than 0*%* and 20*%* or less, which is negative, the microorganism is determined to be an East Asian strain of *Candida auris.*

10. The screening method according to Claim 6, wherein in a case where the enzyme substrate further includes glycerol, and the microorganism has a positive ability to degrade glycerol, the microorganism is determined to be a South American, African, or South Asian strain of *Candida auris.*

11. The screening method according to Claim 6, wherein in a case where the enzyme substrate further includes D-glucosamine hydrochloride, and the microorganism has a negative ability to degrade D-glucosamine hydrochloride, the microorganism is determined to be an East Asian strain of *Candida auris.*

12. The screening method according to Claim 6, wherein in a case where the enzyme substrate further includes D-glucosamine hydrochloride, and the microorganism has an ability to degrade D-glucosamine hydrochloride by more than 80*%* and less than 100*%*, which is positive, the microorganism is determined to be a South American strain of *Candida auris.*

13. The screening method according to Claim 6, wherein in a case where the enzyme substrate further includes D-glucosamine hydrochloride, and the microorganism has a positive ability to degrade D-glucosamine hydrochloride, the microorganism is determined to be an African or South Asian strain of *Candida auris.*

14. The screening method according to Claim 6, wherein in the step of culturing the microorganism, the culture is carried out at 35°C or higher.

## Patentansprüche

1. Verwendung eines Screening-Kulturmediums zum Screening von *Candida auris* basierend auf einer positiven oder negativen Fähigkeit von *Candida auris*, ein Enzymsubstrat abzubauen,
wobei das Medium ein Enzymsubstrat umfasst,
wobei das Enzymsubstrat Raffinose und Xylose umfasst, wobei jedes von Raffinose und
Xylose so gelabelt ist, dass sich im Falle eines Abbaus eine andere Farbe entwickelt, und
wobei bestimmt wird, dass es sich bei dem Mikroorganismus um *Candida auris* handelt, wenn der Mikroorganismus eine positive Fähigkeit zum Abbau von Raffinose und eine negative Fähigkeit zum Abbau von Xylose aufweist.

2. Verwendung eines Screening-Kulturmediums nach Anspruch 1, wobei *Candida auris* ein ostasiatischer Stamm von *Candida auris* ist, und das Enzymsubstrat außerdem N-Acetylglucosamin umfasst.

3. Verwendung eines Screening-Kulturmediums nach Anspruch 1 oder 2, wobei *Candida auris* ein ostasiatischer Stamm von *Candida auris* ist und das Enzymsubstrat außerdem Kaliumgluconat umfasst.

4. Verwendung eines Screening-Kulturmediums nach Anspruch 1 bis 3, wobei das Enzymsubstrat außerdem Glycerin umfasst.

5. Verwendung eines Screening-Kulturmediums nach Anspruch 1 bis 4, wobei das Enzymsubstrat außerdem D-Glucosaminhydrochlorid.

6. Verfahren zum Screening des Mikroorganismus *Candida auris* basierend auf einer positiven oder negativen Fähigkeit von *Candida auris*, ein Enzymsubstrat abzubauen, umfassend:
- einen Schritt des Beimpfens einer Probe, die einen Mikroorganismus enthält, in einem Screening-Kulturmedium, das ein Enzymsubstrat enthält, wobei dieses Enzymsubstrat Raffinose und Xylose umfasst;
- einen Schritt der Kultivierung des in der Probe enthaltenen Mikroorganismus in dem Screening-Kulturmedium; und
- einen Schritt des Screenings von *Candida auris* als den Mikroorganismus durch Nachweis von *Candida auris* im Screening-Kulturmedium;
wobei das Screening-Kulturmedium den Nachweis des Vorhandenseins des Mikroorganismus *Candida auris* ermöglicht, basierend auf der Fähigkeit des Mikroorganismus, das Enzymsubstrat abzubauen, indem es jedem Enzymsubstrat ermöglicht, eine andere Farbe zu entwickeln, und
wobei in einem Fall, in dem der Mikroorganismus eine positive Fähigkeit zum Abbau von Raffinose und eine negative Fähigkeit zum Abbau von Xylose aufweist, bestimmt wird, dass es sich bei dem Mikroorganismus um *Candida auris* handelt.

7. Screening-Verfahren nach Anspruch 6, wobei in einem Fall, in dem das Enzymsubstrat außerdem N-Acetylglucosamin umfasst und der Mikroorganismus eine negative Fähigkeit zum Abbau von N-Acetylglucosamin aufweist, bestimmt wird, dass es sich bei dem Mikroorganismus um einen ostasiatischen Stamm von *Candida auris* handelt.

8. Screening-Verfahren nach Anspruch 6, wobei in einem Fall, in dem das Enzymsubstrat außerdem Kaliumgluconat umfasst und der Mikroorganismus eine negative Fähigkeit zum Abbau von Kaliumgluconat aufweist, bestimmt wird, dass es sich bei dem Mikroorganismus um einen ostasiatischen Stamm von *Candida auris* handelt

9. Screening-Verfahren nach Anspruch 6, wobei in einem Fall, in dem das Enzymsubstrat außerdem Glycerin enthält und der Mikroorganismus die Fähigkeit aufweist, Glycerin um mehr als 0 % und 20 % oder weniger abzubauen, was negativ ist, bestimmt wird, dass es sich bei dem Mikroorganismus um einen ostasiatischen Stamm von *Candida auris* handelt

10. Screening-Verfahren nach Anspruch 6, wobei in einem Fall, in dem das Enzymsubstrat außerdem Glycerin umfasst und der Mikroorganismus eine positive Fähigkeit zum Abbau von Glycerin aufweist, bestimmt wird, dass es sich bei dem Mikroorganismus um einen südamerikanischen, afrikanischen oder südasiatischen Stamm von *Candida auris* handelt.

11. Screening-Verfahren nach Anspruch 6, wobei in einem Fall, in dem das Enzymsubstrat außerdem D-Glucosaminhydrochlorid umfasst und der Mikroorganismus eine negative Fähigkeit zum Abbau von D-Glucosaminhydrochlorid aufweist, bestimmt wird, dass es sich bei dem Mikroorganismus um einen ostasiatischen Stamm von *Candida auris* handelt.

12. Screening-Verfahren nach Anspruch 6, wobei in einem Fall, in dem das Enzymsubstrat außerdem D-Glucosaminhydrochlorid umfasst und der Mikroorganismus die Fähigkeit aufweist, D-Glucosaminhydrochlorid um mehr als 80 % und weniger als 100 % abzubauen, was positiv ist, bestimmt wird, dass es sich bei dem Mikroorganismus um einen südamerikanischen Stamm von *Candida auris* handelt.

13. Screening-Verfahren nach Anspruch 6, wobei in einem Fall, in dem das Enzymsubstrat außerdem D-Glucosaminhydrochlorid umfasst und der Mikroorganismus eine positive Fähigkeit zum Abbau von D-Glucosaminhydrochlorid aufweist, bestimmt wird, dass es sich bei dem Mikroorganismus um einen afrikanischen oder südasiatischen Stamm von *Candida auris* handelt.

14. Screening-Verfahren nach Anspruch 6, wobei im Schritt der Kultivierung des Mikroorganismus die Kultivierung bei 35°C oder höher durchgeführt wird.

## Revendications

1. Utilisation d'un milieu de culture de criblage pour cribler *Candida auris* sur la base de la capacité positive ou négative de *Candida auris* à dégrader un substrat enzymatique,
dans laquelle le milieu comprend un substrat enzymatique,
dans laquelle le substrat enzymatique contient du raffinose et du xylose,
dans laquelle chacun parmi le raffinose et le xylose est marqué de façon à ce qu'une couleur différente soit développée dans le cas où il est dégradé, et
dans laquelle le microorganisme est déterminé être *Candida auris*, dans le cas où le microorganisme a une capacité positive à dégrader le raffinose, et une capacité négative à dégrader le xylose.

2. Utilisation d'un milieu de culture de criblage selon la revendication 1, dans laquelle *Candida auris* est une souche asiatique orientale de *Candida auris*, et le substrat enzymatique contient en outre de la N-acétylglucosamine.

3. Utilisation d'un milieu de culture de criblage selon la revendication 1 ou 2, dans laquelle *Candida auris* est une souche asiatique orientale de *Candida auris*, et le substrat enzymatique contient en outre du gluconate de potassium.

4. Utilisation d'un milieu de culture de criblage selon l'une quelconque des revendications 1 à 3, dans laquelle le substrat enzymatique contient en outre du glycérol.

5. Utilisation d'un milieu de culture de criblage selon l'une quelconque des revendications 1 à 4, dans laquelle le substrat enzymatique contient en outre du chlorhydrate de D-glucosamine.

6. Méthode pour cribler le microorganisme *Candida auris* sur la base d'une capacité positive ou négative de *Candida auris* à dégrader un substrat enzymatique, comprenant :
- une étape d'inoculation d'un échantillon contenant un microorganisme dans un milieu de culture de criblage contenant un substrat enzymatique, ledit substrat enzymatique comprenant du raffinose et du xylose ;
- une étape de culture du microorganisme contenu dans l'échantillon dans le milieu de culture de criblage ; et
- une étape de criblage de *Candida auris* en tant que microorganisme par détection de *Candida auris* dans le milieu de culture de criblage ;
dans laquelle le milieu de culture de criblage permet la détection de la présence du microorganisme *Candida auris* sur la base de la capacité du microorganisme à dégrader le substrat enzymatique, en laissant chaque substrat enzymatique développer une couleur différente, et
dans laquelle le microorganisme est déterminé être *Candida auris*, dans le cas où le microorganisme a une capacité positive à dégrader le raffinose, et une capacité négative à dégrader le xylose.

7. Méthode de criblage selon la revendication 6, dans laquelle, dans le cas où le substrat enzymatique contient en outre de la N-acétylglucosamine, et le microorganisme a une capacité négative à dégrader la N-acétylglucosamine, il est déterminé que le microorganisme est une souche asiatique orientale de *Candida auris.*

8. Méthode de criblage selon la revendication 6, dans laquelle, dans le cas où le substrat enzymatique contient en outre du gluconate de potassium, et le microorganisme a une capacité négative à dégrader le gluconate de potassium, il est déterminé que le microorganisme est une souche asiatique orientale de *Candida auris.*

9. Méthode de criblage selon la revendication 6, dans laquelle, dans le cas où le substrat enzymatique contient en outre du glycérol, et le microorganisme a une capacité à dégrader le glycérol de plus de 0 % et de 20 % ou moins, qui est négative, il est déterminé que le microorganisme est une souche asiatique orientale de *Candida auris.*

10. Méthode de criblage selon la revendication 6, dans laquelle, dans le cas où le substrat enzymatique contient en outre du glycérol, et le microorganisme a une capacité positive à dégrader le glycérol, il est déterminé que le microorganisme est une souche sud-américaine, africaine, ou sud-asiatique de *Candida auris.*

11. Méthode de criblage selon la revendication 6, dans laquelle, dans le cas où le substrat enzymatique contient en outre du chlorhydrate de D-glucosamine, et le microorganisme a une capacité négative à dégrader le chlorhydrate de D-glucosamine, il est déterminé que le microorganisme est une souche asiatique orientale de *Candida auris.*

12. Méthode de criblage selon la revendication 6, dans laquelle, dans le cas où le substrat enzymatique contient en outre du chlorhydrate de D-glucosamine, et le microorganisme a une capacité à dégrader le chlorhydrate de D-glucosamine de plus de 80 % et de moins de 100 %, qui est positive, il est déterminé que le microorganisme est une souche sud-américaine de *Candida auris.*

13. Méthode de criblage selon la revendication 6, dans laquelle, dans le cas où le substrat enzymatique contient en outre du chlorhydrate de D-glucosamine, et le microorganisme a une capacité positive à dégrader le chlorhydrate de D-glucosamine, il est déterminé que le microorganisme est une souche africaine ou sud-asiatique de *Candida auris.*

14. Méthode de criblage selon la revendication 6, dans laquelle, dans l'étape de culture du microorganisme, la culture est effectuée à 35 °C ou plus.
